# EUROPEAN PATENT APPLICATION

(11) **EP 1 364 626 A1**
(43) Date of publication of application: **26.11.2003**
(21) Application number: 03009394.2
(22) Date of filing: 25.04.2003
(51) Int. Cl.: A61F 2/06

(54) **Endovascular stent graft and fixation cuff**

(30) Priority: 25.04.2002 US 133127
(71) Applicant: Medtronic AVE, Inc., Santa Rosa, California 95403 (US)
(72) Inventor: Letort, Michel, 01280 Prevessins (FR)
(74) Representative: Zimmermann, Gerd Heinrich

(57) **Abstract**

An endoluminal prosthesis (210) is provided with an improved fixation system for coupling the endoluminal prosthesis to an inner wall of a lumen. According to an embodiment of the present invention, the functions of securing proximal fixation of of the graft and delivering the main body of the prosthesis to bypass the diseased vessels are independently carried out using a modular device and modular steps for delivery and placement of the modular components. In particular a modular fixation device is provided for initial fixation to a lumen wall and a longer endoluminal prosthesis is provided for coupling to the fixation device, and bypassing a diseased portion of the anatomy. In one embodiment, the longer endoluminal prosthesis includes hook-like members (117) that engage loops on the modular fixation device.

## Description

### FIELD OF THE INVENTION

The present invention relates to tubular prostheses such as grafts and endoluminal prostheses including, for example, stent-grafts and aneurysm exclusion devices, and methods for placement of such grafts and endoluminal structures. In particular, the present invention relates to a modular cuff for providing improved endoluminal prosthesis fixation.

### BACKGROUND OF THE INVENTION

A wide range of medical treatments have been previously developed using "endoluminal prostheses," which terms are herein intended to mean medical devices which are adapted for temporary or permanent implantation within a body lumen, including both naturally occurring or artificially made lumens. Examples of lumens in which endoluminal prostheses may be implanted include, without limitation: arteries such as those located within coronary, mesentery, peripheral, or cerebral vasculature; veins; gastrointestinal tract; biliary tract; urethra; trachea; hepatic shunts; and fallopian tubes. Various types of endoluminal prostheses have also been developed, each providing a uniquely beneficial structure to modify the mechanics of the targeted luminal wall.

A number of vascular devices have been developed for replacing, supplementing or excluding portions of blood vessels. These vascular grafts may include but are not limited to endoluminal vascular prostheses and stent grafts, for example, aneurysm exclusion devices such as abdominal aortic aneurysm ("AAA") devices that are used to exclude aneurysms and provide a prosthetic lumen for the flow of blood. Typically these endoluminal prostheses or stent grafts are constructed of graft materials such as woven polymer materials (e.g., Dacron,) or polytetrafluoroethylene ("PTFE") and a support structure. The stent-grafts typically have graft material such as a woven polymer, secured onto the inner diameter or outer diameter of a support structure that supports the graft material and/or holds it in place against a luminal wall.

One very significant use for endoluminal or vascular prostheses is in treating aneurysms. Vascular aneurysms are the result of abnormal dilation of a blood vessel, usually resulting from disease or a genetic predisposition, which can weaken the arterial wall and allow it to expand. While aneurysms can occur in any blood vessel, most occur in the aorta and peripheral arteries, with the majority of aneurysms occurring in the abdominal aorta. Typically an abdominal aneurysm will begin below the renal arteries and may extend into one or both of the iliac arteries.

Aneurysms, especially abdominal aortic aneurysms, have been treated in open surgery procedures where the diseased vessel segment is bypassed and repaired with an artificial vascular graft. While considered to be an effective surgical technique in view of the alternative of a fatal ruptured abdominal aortic aneurysm, the open surgical technique suffers from a number of disadvantages. The surgical procedure is complex and requires long hospital stays due to serious complications and long recovery times and has high mortality rates. In order to reduce the mortality rates, complications and duration of hospital stays, less invasive devices and techniques have been developed. The improved devices include tubular prostheses that provide a lumen or lumens for blood flow while excluding blood flow to the aneurysm site. They are introduced into the blood vessel using a catheter in a less or minimally invasive technique. Although frequently referred to as stent-grafts, these devices differ from covered stents in that they are not used to mechanically prop open natural blood vessels. Rather, they are used to secure an artificial lumen in a sealing engagement with the vessel wall without further opening the natural blood vessel that is already abnormally dilated.

Most currently used AAA devices comprise a main body portion fixed at the infrarenal aorta junction. The prostheses are typically secured to a vessel wall above and below the aneurysm site with at least one attached expandable annular spring member that provides sufficient radial force so that the prosthesis engages the inner lumen wall of the body lumen to seal the prosthetic lumen from the aneurysm. The devices are typically delivered by initially placing a main body endoluminally and engaging the device to the aorta wall by a series of self-expanding annular spring members. The main body is frequently a bifurcated device with a long and short iliac leg for directing blood flow through the iliac arteries. A contralateral leg is delivered and coupled to the short leg of the bifurcated main body graft. Iliac and/or aortic cuffs then may be delivered if desired to improve or extend deployment or fixation through desired regions.

In general, in many diseased vessels, the area for prosthesis fixation above an aneurysm or other diseased portion may be limited. In addition, the anatomical structure where the graft is to be deployed may curve, twist or be angulated, resulting in poor fixation slipping or kinking, and thus make secure fixation of a long stent graft more difficult. In some devices, super renal fixation is provided to improve fixation. Such fixation requires that the renal arteries not be blocked when the stent graft is deployed. One example of such fixation is an open spring member extending proximally from the graft material. The openings in the spring member permit blood flow so that the renal arteries are not occluded. However, super renal fixation may not address fixation and folding issues presented by highly tortuous anatomy around the aneurysm and iliac vessels. In other devices, other mechanisms have also been used to engage the vessel walls such as, for example, forcibly expandable members or hook like members that puncture the vessel wall.

It would accordingly be desirable to provide a stent graft fixation system that provides improved fixation with a confined or limited area upstream of an aneurysm site. It would also be desirable to provide a device that could increase the area of stent graft fixation. It would also be desirable to provide improved fixation in curved twisted or angulated vessels. It would also be desirable to provide an improved fixation system for an endoluminal prosthesis that reduces trauma to tissue.

Furthermore, in the region surrounding arteries feeding into the vessel, the region for fixation is not always consistent. For example, thrombosis in the region of the aneurysm may cause slippery areas. It would be desirable to provide a device to ensure good proximal fixation of the prosthesis under a variety of conditions.

Another concern is that the prostheses, once deployed, are difficult to remove if not properly secured. If a device is not securely placed or fixed at the infrarenal aortic neck junction, extensions may be added to the prosthesis to provide additional fixation. However, it would be desirable to avoid this situation by providing more predictable, reliable fixation. Accordingly, it would be desirable to provide an improved mechanism that would allow adequate fixation before fully deploying the stent graft

It would also be desirable to provide an improved seal between the aorta and a prosthesis. It would also be desirable to provide a device that would allow a reduction of the initial diameter of a delivery system. It would also be desirable to maintain a consistent outer diameter of a delivery system.

### SUMMARY OF THE INVENTION

An embodiment according to present invention provides an endoluminal prosthesis with an improved fixation system for coupling the endoluminal prosthesis to an inner wall of a lumen. According to an embodiment of the present invention, the functions of securing proximal fixation and delivering the main body of the prosthesis to bypass the diseased vessels are independently carried out using a modular device and modular steps for delivery and placement of the modular components.

In particular a modular fixation device is provided for initial fixation to a lumen wall. Further, a longer endoluminal prosthesis is provided for coupling to the fixation device and bypassing a diseased portion of the anatomy. The fixation device may provide a more reliable landing zone for the prosthesis and a more predictable, consistent engagement area. In one embodiment, the fixation device comprises one or more support structures and a sealing material surrounding at least a portion of a support structure. The fixation device is arranged to engage the inner lumen wall at a fixation site. The fixation device may be a tubular cuff that engages and forms a leak resistant seal with the inner wall of the body lumen. The cuff initially secures the proximal fixation area. Then, when an adequate seal and fixation is made available through the cuff, the main body of the prosthesis is delivered inside the cuff and deployed in a manner that provides fixation and seal between the prosthesis and the cuff.

In one embodiment, the cuff and the endoluminal prosthesis are each constructed of a tubular graft material (such as a woven polymer for conducting fluid) supported by annular spring members. When deployed, annular members of the cuff maintain the cuff, in a conformed, sealing arrangement with the inner wall of the body lumen. Likewise, the annular members of the prosthesis support the tubular graft and maintain the lumen provided by the prosthesis open and in a conformed, sealing arrangement with the inner wall of the cuff, providing a lumen through which body fluids may flow.

The annular support members each comprise an annular expandable member formed by a series of connected compressible diamond structures. Alternatively, for example, the expandable member may be formed of an undulating or sinusoidal-like patterned wire ring or other compressible spring member. Preferably the annular support members are radially compressible springs biased in a radially outward direction, which when released, bias the cuff or the prosthesis into conforming fixed engagement with an interior surface of the vessel or the interior of the cuff respectively. Annular support members are used to create a seal between the cuff and the inner wall of a body lumen, the prosthesis and the cuff, as well as to support the tubular graft structures. The annular springs are preferably constructed of Nitinol. Examples of such annular support structures are described, for example, in U.S. Patent Nos. 5,713,917 and 5,824,041 incorporated herein by reference. When used in an aneurysm exclusion device, the springs have sufficient radial spring force and flexibility to conformingly engage the cuff with the body lumen inner wall and the prosthesis with the cuff, to avoid excessive leakage, and prevent pressurization of the aneurysm, i.e., provide a leak resistant seal. Although some leakage of blood or other body fluid may occur into the aneurysm isolated by the prosthesis, an optimal seal will reduce the chances of aneurysm pressurization and resulting rupture. The annular support members are attached or mechanically coupled to the graft material along the tubular graft by various means, such as, for example, by stitching onto either the inside or outside of the tubular graft.

In one embodiment, the cuff is provided with a textured surface, such as velour, on the outside of the cuff, for better and more intimate fixation, sealing and tissue incorporation. The inner surface of the cuff may also include a textured surface, such as velour, for better fixation with the outer surface of the prosthesis. In one embodiment, the cuff comprises a tubular graft material on the inner and outer diameter supported by a one or more annular support members in between the tubular graft.

The prosthesis may rely on a frictional engagement of the inner circumference of the cuff, and/or the prosthesis or cuff may be provided with an alternative or additional coupling mechanism to reduce the risk of migration of the main body from the cuff. According to one embodiment of the invention, the cuff and/or prosthesis are provided with a coupling mechanism for coupling the cuff and prosthesis together. In one variation of the embodiment, the support structure on the outer circumference of the prosthesis includes a catch mechanism such as a protruding structure for engaging a catch mechanism on the cuff to anchor the prosthesis into the cuff. For example, loops of threads on a transverse axis (e.g. threads of the velour on the inner circumference of the cuff) may be used to catch a protruding structure on the outer circumference of the prosthesis. The protruding structures may, for example be a portion of the sinusoidal structure such as a peak/valley of the annular support member which may protrude from the outer wall of the prosthesis. When inserted into the cuff, the catch mechanism of the prosthesis engages the catch mechanism on the cuff as the prosthesis is pulled in a distal direction within the cuff. Alternative mechanisms for coupling the cuff and the prosthesis may be used. For example, the inner circumference may have protruding structures for catching on the outer circumference of the endoluminal prosthesis.

In one embodiment according to the present invention the tubular graft and fixation device are placed within a blood vessel for the treatment of an aneurysm. According to this embodiment, a cuff is placed above the aneurysm site, e.g., at the infrarenal aortic neck junction above an abdominal aneurysm. The endoluminal prosthesis is fixed in the cuff and is deployed to act as an aneurysm exclusion device forming a lumen for the flow of body fluids excluding the flow at the aneurysm site. The aneurysm exclusion device may be used in other regions such as the thoracic region.

The endoluminal prosthesis may be in the form of either a straight single-limb tubular member or a generally Y-shaped bifurcated tubular member having a trunk joining at a graft junction with a pair of lateral limbs, namely an ipsilateral limb and a contralateral limb. In an abdominal aneurysm, a bifurcated device is frequently preferred. In such a bifurcated prosthesis, the proximal portion of the prosthesis comprises a trunk with a proximal opening, and a distal portion branched into at least two branches with distal openings. Thus, body fluids may flow from the proximal opening through the distal openings of the branches. Preferably the ipsilateral limb is longer so that when deployed, it extends into the common iliac. A single limb extension member is provided having a mating portion for coupling with a lateral limb of a bifurcated member and an adjustable length portion extending coaxially from a distal end of the mating portion.

The compressed profile of the cuff and prosthesis are sufficiently low to allow each of the components to be placed into the vasculature using a low profile delivery catheter. The cuff and prosthesis can be placed within a diseased vessel via deployment means at the location of an aneurysm. Various means for delivery of a prosthesis through the vasculature to the site for deployment, are well known in the art and may be found for example is U.S. Patent Nos. 5,713,917 and 5,824,041. In general, the cuff and endoluminal prosthesis are each radially compressed and loaded in or on the distal end of the catheter for delivery to the deployment site. The aneurysm site is located using an imaging technique such as fluoroscopy, and the catheter is guided through a femoral iliac artery with the use of a guide wire to the aneurysm site. Once appropriately located, a sheath on the catheter covering or restraining the cuff is retracted. The cuff is then released, thus allowing the annular springs to expand and attach or engage the cuff to the inner wall of the body lumen. A distal end of the delivery catheter carrying the endoluminal prosthesis is then placed within the cuff. The proximal portion of the endoluminal prosthesis is similarly deployed within the cuff with the annular support members on the proximal end of the prosthesis expanding to engage the inner circumference of the cuff. If a catch mechanism is used, the prosthesis is maneuvered so that the cuff and prosthesis are further coupled together with the catch mechanism. The endoluminal prosthesis affixed to the cuff is further deployed to bypass the aneurysm or other diseased portion of the vessel. The iliac extension is also loaded into a catheter and is then located into the main body of the stent graft and within the iliac vessel where it is deployed. When deployed, the iliac extension is engaged using annular springs at the proximal end within the inner lumen of the main body and at the distal end within the inner wall of the iliac vessel.

These and further aspects of the invention are exemplified and in the detailed description of embodiments according to the invention described below.

### BRIEF DESCRIPTION OF THE DRAWINGS

Figure 1 is a side perspective view of a fixation cuff according to the invention
Figure 2 is a cross sectional view of the cuff of Figure 1 taken along 2-2.
Figure 3 is a side view of an endoluminal prosthesis according to the invention.
Figure 4 is an enlarged side view of a portion of the endoluminal prosthesis of Figure 3.
Figure 5 is a side view and partial cross section of a cuff placed in a diseased abdominal aorta.
Figure 6 is a side view and partial cross section of an endoluminal prosthesis in place in the cuff in the diseased aorta of Figure 6.
Figure 7 is an enlarged view of Figure 6 of a portion of the cuff and prosthesis being positioned together.
Figure 8 is an enlarged view of the portion of the cuff and prosthesis of Figure 7 as the prosthesis is drawn distally to engage the coupling mechanism to position the devices as illustrated in Figure 6.

### DETAILED DESCRIPTION

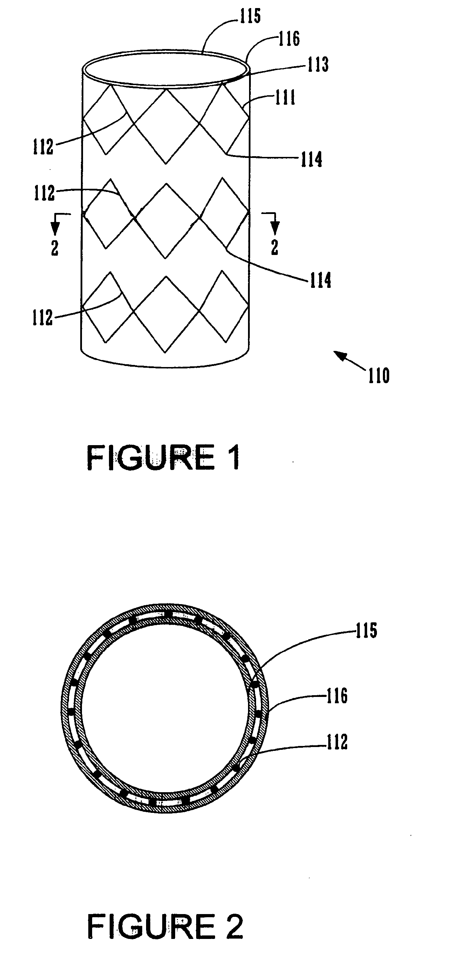

Figures 1-8 illustrate various embodiments of a modular fixation cuff and endoluminal prosthesis, a delivery system and a method according to the present invention. Although a fixation cuff, endoluminal prosthesis, delivery system and method according to the invention may be used in any body lumen that conducts body fluid and may be used as a single lumen prosthesis or a multibranch prosthesis, they are described herein with reference to treatment of an aortic aneurysm, in particular in the abdomen of a patient using a bifurcated prosthesis.

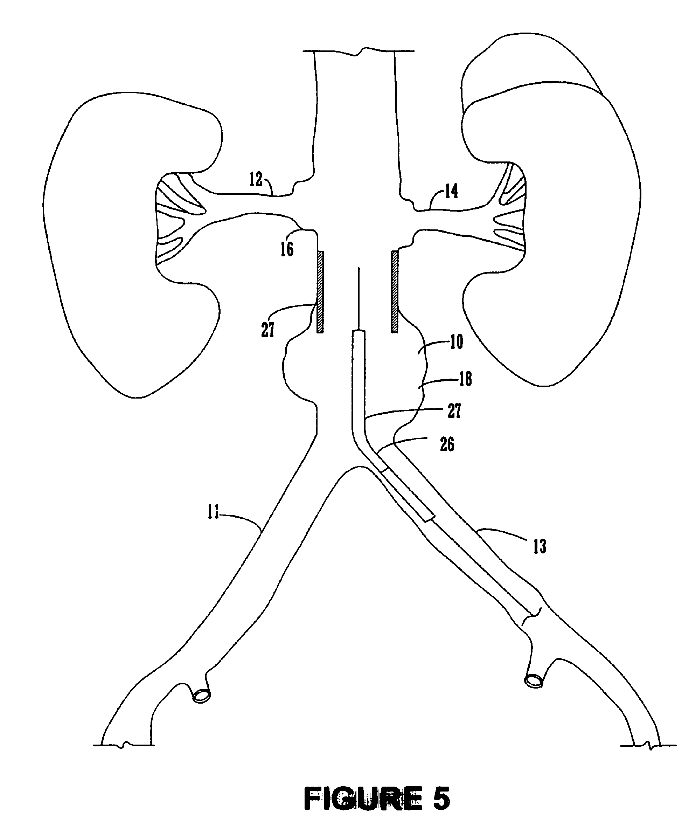

Figures 1 and 2 illustrate an embodiment of a fixation cuff according to the present invention. The cuff 110 comprises an inner tubular graft layer 115 and an outer tubular graft layer 116 and a series of radially compressible annular support members 112 sandwiched between inner and outer tubular graft layers 115, 116. The support members are attached to the tubular graft layers 115, 116 using sutures (not shown) or other coupling means. The cuff 110 is generally between about 5 and 35 mm in length so that it may be placed below the aorta renal junction and above the aortic aneurysm in an area where the cuff 110 may be secured (at the infrarenal aortic neck junction). The annular support members 112 are preferably spring members having predetermined radii and are preferably constructed of a material such as Nitinol in a superelastic, shape set annealed condition. The circumferences of the annular support members 112 comprise a series of connected diamond structures 111. The diamond structures 111 have peaks 113 and valleys 114. Other annular support structures may be used as well, such as a sinusoidal or undulating wire spring member. In Figures 1 and 2, the annular support members 112 are shown in an expanded configuration. Prior to deployment the annular members 112 are compressed. The annular members 112 are configured to support the cuff and 110 and/or bias the cuff 110 into conforming fixed engagement with the inner wall of the aorta 10 just below the aorta-renal junction 16 (Figure 5).

The inner tubular graft 115 is preferably formed of a biocompatible material with a textured inner surface such as velour so that it engages the outer circumference of a prosthesis fixed to the cuff 110 and provides an improved seal. The outer tubular graft 116 is preferably formed of a textured surface such as velour to engage the inner wall of the aorta 10 (Figure 5) and provides an improved seal. The velour used on the inner and/or outer surfaces of the cuff is also a low porosity woven fabric to provide a leak resistant seal. The graft material is relatively thin-walled so that it may be compressed into a small diameter, yet capable of acting as a strong, leak-resistant, fluid conduit when in tubular form. The velour cuff also may allow a reduction of the initial diameter of the delivery system and/or maintain a consistent given outer diameter of the delivery system.

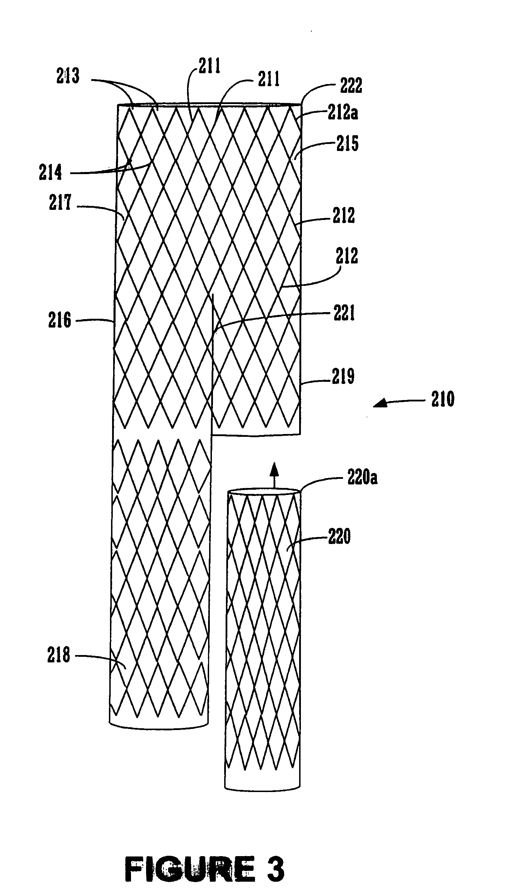

Figure 3 illustrates an endoluminal prosthesis 210. The prosthesis 210 comprises a tubular graft 215 and a series of radially compressible annular support members 212 attached to tubular graft 215. In Figure 3, the annular support members 212 are shown in an expanded configuration. Prior to deployment the annular members 212 are compressed. The annular members 212 support the graft and/or bias the prosthesis 210 into conforming fixed engagement with an interior surface of the cuff 110 (Figures 1 and 2). The annular support members 212 are preferably spring members having predetermined radii and are preferably constructed of a material such as Nitinol in a superelastic, shape set annealed condition.

The tubular graft 215 is preferably formed of a biocompatible, low-porosity woven fabric, such as a woven polyester. The graft material is thin-walled so that it may be compressed into a small diameter, yet capable of acting as a strong, leak-resistant, fluid conduit when in tubular form. In this embodiment, the annular support members 212 are sewn on to the outside of the tubular graft 215 material by sutures. Alternative mechanisms of attachment may be used and the annular support members 212 may be attached to the inside of the tubular graft 215. The support members 212 comprise a series of connected diamond structures 211 around the circumference of the annular member 212 that form peaks 213 and valleys 214.

The prosthesis 210 includes a main body portion 216 and a contralateral iliac extension limb 220. The main body portion 216 is a tubular bifurcated member having has an aortic portion 217, a long ipsilateral iliac limb portion 218, and a short iliac portion 219.

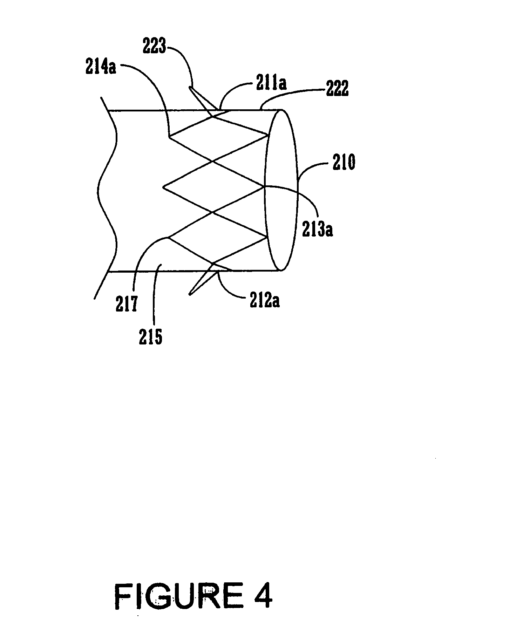

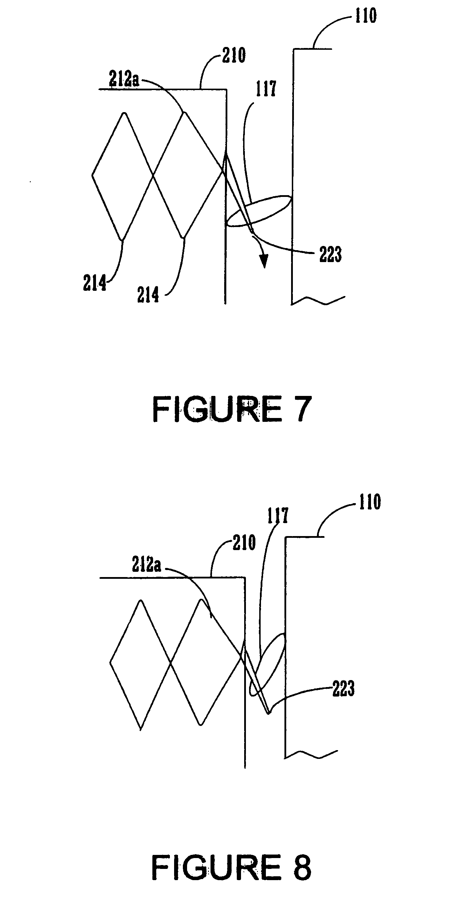

Figure 4 illustrates a enlarged view of the proximal most annular member 212a of the prosthesis 210. The diamond structures 211a around the circumference of the annular member 212a form peaks 213a and valleys 214a where the valleys 214a form protrusions 223 extending in a radial direction from the tubular graft 215. The protrusions 223 (hooks, catches or fixation mechanism) act to engage the cuff 110 as illustrated in Figures 7 and 8.

Referring to Figure 5, the cuff 110 is illustrated in position in an aorta 10 after being deployed by catheter 26. The aorta 10 is joined by renal arteries 12 and 14 at the aorto-renal junction 16. Just below the aorta-renal junction 16 is an aneurysm 18, a diseased region where the vessel wall is weakened and expanded. Below the aneurysm 18, the aorta 10 bifurcates into right and left iliac vessels 11, 13, respectively. Between the aorta-renal junction 16 and the aneurysm 18 is a region of the aorta 10 where the cuff 110 is positioned to be engaged with the inner wall of the aorta 10. Annular support members 112 of the cuff 110 are designed to exert a radially outward force sufficient to bias the cuff 110 into conforming fixed engagement with the inner wall of the aorta above aneurysm 18 to support the inner and outer tubular grafts 115, 116 and to provide a leak resistant seal between the cuff 110 and the inner wall of the aorta 10. The cuff 110 may be slightly longer or shorter than the area between the aorta-renal junction 16 and the aneurysm 18, which varies from patient to patient. As such, the cuff 110 provides more predictable area for deploying the endoluminal prosthesis. Where the potential prosthesis fixation area is limited or inconsistent, the cuff 110 may provide a larger, more consistent area for securing the prosthesis 210. In addition where the prosthesis 210 is deployed in a highly curved or angulated vasculature, the cuff 110 may allow a more secure fixation and compensate for angulation.

To deploy the cuff 110, it is loaded into a catheter 26 in a collapsed position. Annular members 112 are held in a radially compressed position by a sheath or cover 27 placed over the cuff 110 to facilitate its delivery. The cuff 110 is delivered in a compressed state via catheter 26 through a surgically accessed femoral artery, to the desired deployment site below the aorta-renal junction 16. The sheath 27 is retracted when the distal end of the catheter 26 is located at the deployment site within the cuff 110 releasing the annular members 112 from the compressed position to an expanded position engaging the inner wall of the aorta 10.

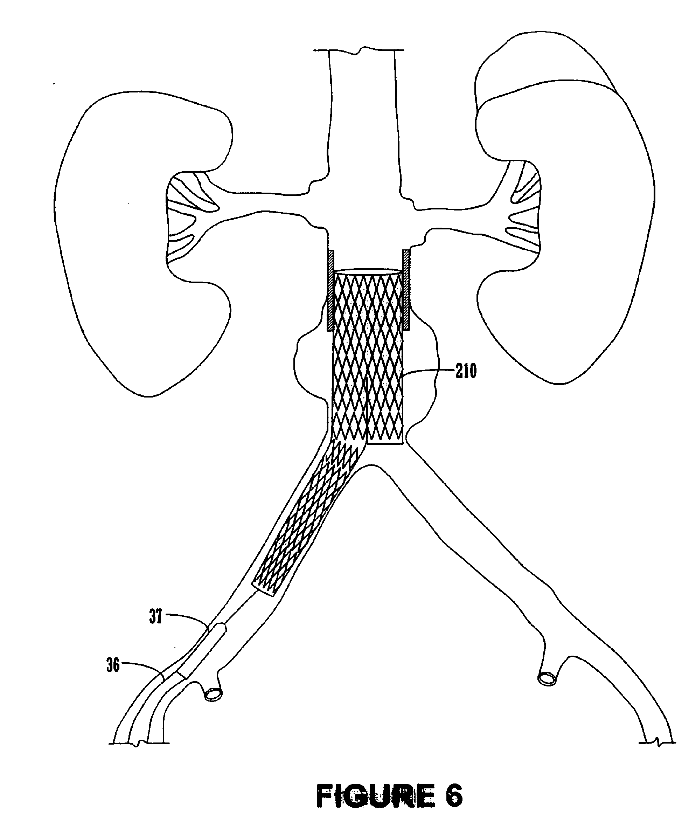

Referring to Figure 6, the prosthesis 210 is shown after it has been deployed within cuff 110 using catheter 36. The proximal end 222 of the prosthesis 210 is placed within the cuff 110, which is in position below the aorta-renal junction 16 in the abdominal aorta 10 as illustrated in Figure 5. Annular support members 212 are designed to exert a radially outward force sufficient to bias the tubular graft 215 of the endoluminal prosthesis 210 into conforming fixed engagement with the interior surface of the cuff 110 to support the tubular graft 215, and/or to provide a leak resistant seal between the prosthesis 210 and the cuff 110 which provides a seal between itself and the inner wall of the aorta 10. The proximal aortic portion 222 of the prosthesis 210 is located within cuff 110, and the long ipsilateral iliac portion limb 218 is located within the right iliac vessel 11. After deployment of the main body portion 216, the contralateral iliac extension limb 220 is located within left iliac vessel 13, and near the graft junction 221 within the short iliac portion 219. The proximal end 220a of the contralateral iliac extension limb 220 includes a proximal support member 212a biasing the proximal end 220a into conforming fixed engagement with the interior surface the short iliac portion 219.

To deploy the prosthesis 210, the main body portion 216 of the prosthesis is loaded into a catheter 36. The prosthesis 210 is loaded in a collapsed position into the catheter 36 where a cover or sheath 37 placed over the prosthesis 210 holds the annular members 212 in a radially compressed position. The main body portion 216 is delivered in a compressed state via catheter 36 through a surgically accessed femoral artery, to the desired deployment site. The sheath 37 is retracted when the distal end of the catheter 36 is located at the deployment site within the cuff 110. The annular members 212 are then released from the compressed position to an expanded configuration. The proximal annular members engage the inner wall of the cuff 110 while the remaining portion extends distally through the aorta 10 beyond the aneurysm 18 with the long ipsilateral limb portion 218 extending into the right iliac vessel 11.

Using a second catheter, the contralateral iliac extension limb 220 may be separately deployed through a surgically accessed femoral artery through the left iliac vessel 13 after placement of the main body portion 216. The proximal end 220a of the contralateral iliac extension limb 220 is located within the short iliac portion 219 of the main body 216 and is similarly released from a delivery catheter. The annular members 212 of the extension limb's proximal end 220a bias the proximal end 220a into conforming fixed engagement with the interior surface the short iliac portion 219.

Referring to Figures 7 and 8 an additional engagement mechanism for coupling the cuff 110 and prosthesis 210 is illustrated. Protrusions 223 from the annular support member 212a of the prosthesis 210 engage loops 117 in (or on the surface of) the material of the inner tubular graft 115 of the cuff 110 as the prosthesis 210 is pulled distally through the cuff 110 to position the prosthesis. As illustrated in Figure 8, as the prosthesis is further pulled distally, the protrusions 223 further engage the loops 117 to reduce the chance of separation of the prosthesis 210 from the cuff 110.

Various structures or other fixation mechanisms may be used, for example hooks, other catches, barbs or endo-staples(pre-fixed to the top of the main body 216) may be used to fix the cuff 110 and the prosthesis 210 together. The hooks, other catches barbs, staples etc. may be located on either or both of the cuff 110 and/or the prosthesis 210.

Surgical methods and apparatus for accessing the surgical site are generally known in the art and may be used to place the catheter within the vasculature and deliver the cuff or prosthesis to the deployment site. The cuff or prosthesis may be delivered to the deployment site by one of several ways. A surgical cut down may be made to access a femoral iliac artery. The catheter is then inserted into the artery and guided to the aneurysm site using fluoroscopic imaging where the device is then deployed. The members supporting the cuff or prosthesis, biased in a radially outward direction, are released to expand and engage the cuff or prosthesis in the vessel against the inner lumen wall or fixation cuff respectively, to provide proximal fixation or an artificial lumen for the flow of blood respectively. Another technique includes percutaneously accessing the blood vessel for catheter delivery, i.e., without a surgical cutdown. An example of such a technique is set forth in U.S. Patent No.5, 713,917, incorporated herein by reference.

Although this detailed description sets forth particular embodiments according to the invention, various other vascular grafts, endoluminal prostheses, and delivery systems are contemplated, for example, other endoluminal prostheses, single lumen grafts, thoracic stent-grafts and other endoluminal prostheses where additional fixation is desired, particularly where separating the steps of fixation and deployment of the prosthesis is desired.

While the invention has been described with reference to particular embodiments, it will be understood to one skilled in the art that variations and modifications may be made in form and detail without departing from the spirit and scope of the invention.

## Claims

1. A modular endoluminal prosthesis (210) comprising:
a) a fixation member (110) comprising a tubular member (115,116) having an opening and including a coupling mechanism configured to couple the fixation member to the inner wall of a body lumen and to form a leak resistant seal therewith;
b) an elongate prosthesis comprising:
a tubular graft (215) comprising a graft material forming a lumen for the flow of body fluid therethrough;
at least one annular support member (212) coupled to the graft material for supporting the graft material to provide a lumen for the flow of body fluid therethrough; and
an engaging member (223) configured to couple the elongate prosthesis to the fixation member,
wherein a portion of the elongate prosthesis is configured to fit within the opening in the fixation member and to engage the fixation member to form a leak resistant seal therewith.

2. The modular endoluminal prosthesis of claim 1 wherein the engaging member comprises the at least one annular support member (212).

3. The modular endoluminal prosthesis of claim 1 or 2 wherein the engaging member comprises a catch mechanism (117,223) configured to couple the elongate prosthesis to the fixation member.

4. The modular endoluminal prosthesis of claim 3 wherein at least a portion of an annular support member protrudes from the tubular member and wherein said catch mechanism comprises said at least a portion of the annular support member.

5. The modular endoluminal prosthesis of any of claims 1 to 4 wherein the fixation member further comprises a catch mechanism configured to couple the fixation member to the elongate prosthesis.

6. The modular prosthesis of claim 5 wherein the tubular member of the fixation member has an inner circumference comprising a graft material and wherein the catch mechanism comprises the graft material.

7. The modular prosthesis of claim 6 wherein the catch mechanism comprises a loop (117) in the graft material.

8. A cuff (110) for fixing an endoluminal prosthesis to the inner wall of a body lumen, said cuff comprising:
a tubular member (115,116) having an opening and including a fixation mechanism configured to fixably engage the cuff to the inner wall of a body lumen and to form a leak resistant seal with the inner wall of the body lumen, wherein the opening in the tubular member is configured to receive an endoluminal prosthesis in a substantially sealing arrangement therewith; and
a coupling member configured to engage a portion of the endoluminal prosthesis to couple the cuff to the endoluminal prosthesis.

9. The cuff of claim 8 wherein the fixation mechanism comprises a self-expanding annular support member configured to hold the cuff in a leak resistant sealing relationship with the inner lumen wall.

10. The cuff of claim 8 or 9 wherein tubular member includes an outer circumference comprising a graft material.

11. The cuff of any of claims 8 to 10 wherein the coupling member comprises a catch mechanism.

12. The cuff of claim 11 wherein the tubular member has an inner circumference comprising a graft material and wherein the catch mechanism comprises a portion of the graft material.

13. The cuff of claim 12 wherein the catch mechanism comprises a loop (117) in the graft material.

14. A method for excluding a diseased portion of a body lumen from the flow of body fluid comprising the steps of:
providing a tubular cuff having an opening therethrough;
fixing the tubular cuff to the inner wall of the body lumen at a fixation site within a body lumen at a location upstream of the diseased portion;
providing an endoluminal prosthesis comprising a tubular member having an proximal opening and a distal opening, said tubular member, proximal opening and distal opening forming a lumen for the flow of body fluid therethrough. Said endoluminal prosthesis comprising an engaging member configured to engage the tubular cuff;
locating a portion of the endoluminal prosthesis within the tubular cuff;
fixing the portion of the endoluminal prosthesis to the tubular cuff by coupling the endoluminal prosthesis to the cuff with the engaging member; and
deploying the endoluminal prosthesis in the body lumen to bypass the diseased portion of the body lumen and to provide a lumen for the flow of body fluid therethrough.

15. The method of claim 14 wherein the step of providing a tubular cuff comprises providing a tubular cuff with a catch, wherein the step of fixing the endoluminal prosthesis comprises engaging the catch of the cuff with the engaging member of the endoluminal prosthesis.

16. The method of claim 15 wherein the step of providing the tubular cuff comprises providing a tubular graft material; and wherein the step of providing the tubular cuff with a catch comprises providing the tubular graft material with a loop in the graft material configured to engage the engaging member of the endoluminal prosthesis.

17. A modular endoluminal prosthesis comprising:
a) a modular fixation means for fixing a prosthesis above an aneurysm within a body lumen, the fixation means comprising a tubular means having an opening and including a coupling means for coupling the fixation means to the inner wall of a body lumen and for forming a leak resistant seal with the inner wall of the body lumen; and
b) an elongate prosthesis means comprising:
a tubular graft means for providing a lumen for the flow of body fluid therethrough;
at least one annular support means coupled to the graft means, said support means for supporting the graft material to provide the lumen for the flow of body fluid therethrough,
wherein a portion of the elongate prosthesis means is configured to fit within the opening in the fixation means and to engage the fixation means to form a leak resistant seal therewith.
